# EUROPÄISCHE PATENTSCHRIFT

(11) **EP 1 385 617 B1**
(45) Veröffentlichungstag und Bekanntmachung des Hinweises auf die Patenterteilung: **12.12.2007**
(21) Anmeldenummer: 02738058.3
(22) Anmeldetag: 10.05.2002
(51) Int. Cl.: B01J 19/00

(54) **HYBRIDVERFAHREN ZUR HERSTELLUNG VON TRÄGERN FÜR DIE ANALYTBESTIMMUNG**
HYBRID METHOD FOR THE PRODUCTION OF CARRIERS FOR ANALYTE DETERMINATION
PROCEDES HYBRIDES PERMETTANT DE PRODUIRE DES SUPPORTS CON US POUR LA RECHERCHE DE SUBSTANCES A ANALYSER

(30) Priorität: 09.05.2001 DE 10122357
(43) Veröffentlichungstag der Anmeldung: 04.02.2004
(73) Patentinhaber: Febit Biotech GmbH, 69120 Heidelberg (DE)
(72) Erfinder: STÄHLER, Cord, F., 69469 Weinheim (DE); STÄHLER, Peer, F., 68167 Mannheim (DE); BEIER, Markus, 69121 Heidelberg (DE); WIXMERTEN, Anke, 64572 Büttelborn (DE); MAURITZ, Ralf, 60435 Frankfurt am Main (DE); SCHLAUERSBACH, Andrea, 63739 Aschaffenburg (DE)
(74) Vertreter: Weiss, Wolfgang
(86) Internationale Anmeldenummer: PCT/EP2002/005179
(87) Internationale Veröffentlichungsnummer: WO 2002/089971

(56) Entgegenhaltungen:
- WO-A-97/19958
- DE-A- 19 940 749
- US-A- 5 196 566
- US-A- 5 770 358
- JEFFREY A. BORGIA & GREGG B. FIELDS: "Chemical Synthesis of Proteins" TRENDS IN BIOTECHNOLOGY - TIBTECH, Bd. 18, Nr. 6, Juni 2000 (2000-06), Seiten 243-251, XP002223565 ELSEVIER PUBLICATIONS, CAMBRIDGE., GB ISSN: 0167-7799

## Beschreibung

Die Erfindung betrifft ein Verfahren und eine Vorrichtung zur Herstellung eines Trägers, insbesondere eines mikrofluidischen Trägers, für die Bestimmung von Analyten.

In den letzten Jahren wurde mit der Technologie von auf einem Träger immobilisierten Rezeptorarrays, z.B. DNA-Chips, ein wertvolles Mittel geschaffen, das die schnelle und hochparallele Durchführung komplexer Analytbestimmungen erlaubt. Das den Rezeptorarrays zugrunde liegende biophysikalische Prinzip ist das der Wechselwirkung eines spezifischen immobilisierten Rezeptors mit einem in einer Flüssigphase vorhandenen Analyten, beispielsweise durch Nukleinsäurehybridisierung, wobei auf verschiedenen Bereichen des Trägers eine Vielzahl von Rezeptoren, z.B. Hybridisierungssonden, angebracht sind, die jeweils mit verschiedenen in der Probe vorhandenen Analyten, z.B. komplementären Nukleinsäureanalyten, spezifisch binden.

Um mit Rezeptorarrays, z.B. mit DNA-Chips, komplexe biologische Fragestellungen, wie Genexpressionsstudien, Targetvalidierung, Sequenzierungen oder Resequenzierungen bearbeiten zu können, ist eine effiziente Herstellung von Rezeptorarrays in hoher Qualität von grundlegender Bedeutung. Hierzu kann neben der Spotting-Technologie (Cheung et al., Nature Genet. Suppl. 1999, Vol. 21, 15-19) die Herstellung von DNA-Arrays auch in situ unter Verwendung von Phosphoramidit-Synthesebausteinen (Caruthers et al., Tetrahedron Lett., 1981, 1859) erfolgen. Hierbei lässt sich zwischen nasschemischen Verfahren (Maskos et al., Nucleic Acids Res. 1992, Vol. 20, 1679-1984) und photochemischen Verfahren (Pease, Proc. Natl. Acad. Sci., 1994, Vol. 91, 5022-5026) unterscheiden.

Ein Träger und ein Verfahren zur Analytbestimmung, die eine integrierte Rezeptorsynthese und Analyse erlauben, sind z.B. in WO 00/13018 beschrieben. Dort erfolgt die Rezeptorsynthese vorzugsweise unter Verwendung von photoaktivierbaren Rezeptorbausteinen. Alternativ wird eine Synthese der Rezeptorbausteine auch durch nasschemische Methoden offenbart.

Eine Variante der photochemischen Verfahren ist in US 6,022,963 offenbart. Diese Schrift befasst sich mit neuen photochemischen Schutzgruppen. Es werden neue photochemische Verbindungen und die prinzipielle Möglichkeit beschrieben, dass diese photochemischen Schutzgruppen auch durch nasschemische Schritte abzuspalten sein können. In einer Variante dazu wird das Verfahren dahingehend abgewandelt, dass zunächst nasschemische Schutzgruppen an den Rezeptorbausteinen vorgegeben sind, und diese dann abgespalten werden, um sie auf dem gesamten Träger in situ durch photochemische Schutzgruppen zu ersetzen. Daran schließt sich dann wieder ein photochemischer Schritt an, der die gewünschte ortsaufgelöste Synthese ermöglicht. Eine Kombination von photochemischen und nasschemischen Rezeptorbausteinen wird nicht offenbart.

Bei nasschemischen Verfahren werden Rezeptorbausteine, z.B. Phosphoramidit-Nukleotidbausteine, eingesetzt, die eine temporäre nasschemische Schutzgruppe an der 3'- oder/und 5'-Position tragen. Diese Schutzgruppe wird durch einen nasschemischen Schritt abgespalten, z.B. im Falle der gebräuchlichen Dimethoxytrityl-Schutzgruppe durch Säurebehandlung, beispielsweise mit Trichloressigsäure. Problematisch ist, dass die Anwendung des Deprotektionsmediums (Säure) flächig erfolgt, so dass eine ortsaufgelöste Abspaltung nur sehr schwierig möglich ist und kaum individuelle Bereiche auf dem Träger angesprochen werden können. Vorteilhaft ist jedoch, dass die Abstraktion der temporären Schutzgruppe durch nasschemische Methoden und die Kondensation des nächsten Synthesebausteins mit hoher Effizienz (ca. ≥ 98-99 %) verläuft.

Bei photochemischen Verfahren werden Rezeptorbausteine, z.B. Phosphoramidit-Nukleotidbausteine eingesetzt, die eine temporäre photochemische Schutzgruppe an 3'- oder/und 5'-Position tragen. Diese Schutzgruppe wird in einem photochemischen Schritt abgespalten. Dies kann durch ortsspezifische Belichtung des Bereichs erfolgen, an dem die Photoschutzgruppe abgespalten und dann anschließend eine Fortsetzung der Rezeptorsyntheses erfolgen soll. Hierbei gilt, dass die Applikation des Deprotektionsmediums (Licht) nicht flächig, sondern ortsaufgelöst erfolgen kann, z.B. durch Verwendung einer Maske oder durch eine programmierbare Lichtquelle (siehe z.B. WO 00/13018). Somit können individuelle Syntheseorte angesprochen werden. Nachteilig erweist sich jedoch, dass die Abspaltung der temporären Photoschutzgruppe mit vergleichsweise geringer Effizienz (ca. 90-95 %) verläuft und somit die Synthesequalität und Gesamtausbeute im Vergleich zu nasschemischen Verfahren sinkt.

Eine Aufgabe der vorliegenden Erfindung war es, ein System zur Herstellung eines Trägers für die Analytbestimmung bereitzustellen, bei dem die Nachteile des Standes der Technik mindestens teilweise vermieden werden.

Diese Aufgabe wird durch ein Verfahren gelöst, welches die Vorteile nasschemischer und photochemischer Prozesse vereint. Aus dem gezielten Zusammenwirken dieser beiden Methoden ergeben sich neue Strategien zum Aufbau und zur Anwendung von Rezeptorarrays, z.B. DNA-Chips, wie etwa Arrays mit mehreren verschiedenen Rezeptorsequenzen pro Stellplatz bzw. Bereich, die bislang durch eine der beiden Methoden allein nicht möglich waren.

Beim erfindungsgemäßen Hybridverfahren werden sowohl nasschemische als auch photochemische Rezeptorbausteine, z.B. Phosphoramiditbausteine, eingesetzt. Zur Optimierung des Syntheseweges kann ein spezifischer Algorithmus eingesetzt werden, der sicherstellt, dass die auf dem Array zu synthetisierenden Rezeptoren möglichst schnell und in hoher Qualität aufgebaut werden können. Über die Verwendung von zwei verschiedenen Rezeptorbausteinen hinaus, z.B. photolabilen und säurelabilen Rezeptorbausteinen, lässt sich der Algorithmus unter Verwendung weiterer Schutzgruppen, z.B. ortsauflösender Schutzgruppen, wie etwa elektrochemischer Schutzgruppen oder/und anderer nasschemischer Schutzgruppen (z.B. basenlabiler oder oxidationslabiler Schutzgruppen), erweitern, so dass die Flexibilität des Systems noch weiter gesteigert wird.

Ein Gegenstand der vorliegenden Erfindung ist somit ein Verfahren zur Herstellung eines Trägers für die Bestimmung von Analyten, umfassend die Schritte:
(a) Bereitstellen eines Trägers,
(b) Leiten von Flüssigkeit mit Bausteinen für die Synthese polymerer Rezeptoren über den Träger,
(c) orts- oder/und zeitspezifisches Immobilisieren der Rezeptorbausteine an jeweils vorbestimmten Bereichen auf dem Träger und
(d) Wiederholen der Schritte (b) und (c) bis die gewünschten Rezeptoren an den jeweils vorbestimmten Bereichen synthetisiert worden sind,
dadurch gekennzeichnet, dass die Synthese der Rezeptoren eine Kombination von nasschemischen und photochemischen Syntheseschritten umfasst und dass man einen Träger mit mehreren verschiedenen Rezeptorbereichen herstellt.

Die vorliegende Erfindung zeichnet sich insbesondere dadurch aus, dass das Verfahren zur Herstellung des Trägers mit einem Detektionssystem zur Analytbestimmung integriert werden kann. Dieses Detektionssystem kann zur integrierten Synthese und Analyse eingesetzt werden, insbesondere zum Aufbau komplexer Träger, z.B. Biochips, und zur Analyse komplexer Proben, z.B. zur Genom-, Genexpressions- oder Proteomanalyse.

Die Synthese der Rezeptoren erfolgt in situ auf dem Träger, beispielsweise indem Fluid mit Rezeptorsynthesebausteinen über den Träger geleitet wird, die Bausteine an den jeweils vorbestimmten Bereichen auf dem Träger ortsoder/und zeitspezifisch immobilisiert werden und diese Schritte wiederholt werden, bis die gewünschten Rezeptoren an den jeweils vorbestimmten Bereichen auf dem Träger synthetisiert worden sind. Ein wesentliches Merkmal der erfindungsgemäßen Rezeptorsynthese besteht darin, dass mindestens ein nasschemischer Syntheseschritt und mindestens ein photochemischer Syntheseschrittmiteinander kombiniert werden. Weiterhin umfasst die Rezeptorsynthese vorzugsweise eine online-Prozessüberwachung, um eine ausreichende Qualität der auf dem Array immobilisierten Rezeptoren zu gewährleisten.

Der durch das erfindungsgemäße Verfahren hergestellte Träger ist vorzugsweise in einer Vorrichtung zur Bestimmung von Analyten integriert, umfassend
(i) eine Lichtquellenmatrix, vorzugweise eine programmierbare Lichtquellenmatrix, z.B, ausgewählt aus einer Lichtventilmatrix, einem Spiegelarray und einem UV-Laserarray,
(ii) einen Träger, vorzugweise einen mikrofluidischen Träger mit Kanälen, insbesondere mit geschlossenen Kanälen, in denen sich die vorbestimmten Bereiche mit den jeweils unterschiedlich immobilisierten Rezeptoren befinden, wobei die Kanäle vorzugsweise im Bereich von 10 µm bis 10000 µm, besonders bevorzugt im Bereich von 50 bis 250 µm, liegen und grundsätzlich in beliebiger Form ausgestaltet sein können, z.B. mit rundem, ovalem, quadratischem oder rechteckigem Querschnitt,
(iii) Mittel zur Zufuhr von Fluid zum Träger und zur Ableitung von Fluid aus dem Träger und
(iv) eine Detektionsmatrix, z.B. eine optische Detektionsmatrix, wie etwa eine CCD-Matrix oder/und eine elektronische Detektionsmatrix, wie sie in WO 00/13018 beschrieben ist.

In einer bevorzugten Ausführungsform bietet der Träger durch eine Einteilung in fluidische Subräume, die getrennt voneinander adressiert werden können, die Möglichkeit, die ortsspezifische Immobilisierung zu bestimmen. Ein Träger, der dieses Kriterium erfüllt, ist in WO 00/13018 beschrieben. Der Träger bietet dabei die Aufteilung der reaktiven Bereiche in 2 oder mehr Subräume.

Die Rezeptoren werden vorzugsweise ausgewählt aus Biopolymeren, die in situ auf dem Träger aus den entsprechenden Synthesebausteinen durch eine Kombination lichtgesteuerter und nasschemischer Prozesse sythetisiert werden können. Als Synthesebausteine können dabei sowohl monomere, z.B. Mononukleotide, Aminosäuren etc., als auch oligomere Bausteine, z.B. Di-, Tri- oder Tetranukleotide, Di-, Tri- oder Tetrapeptide etc., eingesetzt werden. Bevorzugt werden die Rezeptoren ausgewählt aus Nukleinsäuren wie DNA, RNA, Nukleinsäureanaloga wie Peptidnukleinsäuren (PNA), Proteinen, Peptiden und Kohlenhydraten. Besonders bevorzugt werden die Rezeptoren aus Nukleinsäuren und Nukleinsäureanaloga ausgewählt und in einem Nachweisverfahren zur Hybridisierung von komplementären Nukleinsäureanalyten eingesetzt.

Die Rezeptorsynthese umfasst vorzugsweise die Verwendung von Synthesebausteinen mit nasschemischen Schutzgruppen und von Rezeptorbausteinen mit photochemischen Schutzgruppen. Gegebenenfalls können auch Synthesebausteine verwendet werden, die sowohl nasschemische als auch photochemische Schutzgruppen oder Hybridschutzgruppen, d.h. Gruppen, die zweistufig durch einen nasschemischen und eine photochemischen Schritt abspaltbar sind, tragen. Beispiele für nasschemische Schutzgruppen sind beliebige Schutzgruppen, wie aus dem Stand der Technik zur Synthese von Biopolymeren, wie etwa Nukleinsäuren oder Peptiden, auf festen Trägern bekannt sind. Bevorzugte Beispiele sind säurelabile Schutzgruppen, basenlabile Schutzgruppen, gegenüber Oxidation labile Schutzgruppen oder enzymatisch abspaltbare Schutzgruppen. Die Verwendung von säurelabilen Schutzgruppen, wie etwa Dimethoxytrityl, ist besonders bevorzugt. Für die photochemischen Syntheseschritte können beliebige photochemische Schutzgruppen, wie sie aus dem Stand der Technik zur Synthese von Biopolymeren, wie etwa Nukleinsäuren oder Peptiden, auf festen Trägern bekannt sind, eingesetzt werden. Bevorzugte Beispiele für photochemische Schutzgruppen sind in DE 101 05 079.8 und bevorzugte Beispiele für Hybridschutzgruppen sind in DE 101 05 077.1 beschrieben.

Weitere bevorzugte Schutzgruppen sind sogenannte zweistufige Schutzgruppen, die durch einen Belichtungsschritt aktiviert und anschließend durch einen chemischen Behandlungsschritt gespalten werden. Der chemische Behandlungsschritt umfasst vorzugsweise eine Behandlung mit Base, eine Behandlung mit Säure, eine Oxidation, eine Reduktion oder/und eine enzymatische Reaktion. Besonders bevorzugt sind derivatisierte Tritylgruppen als zweistufige Schutzgruppen, wie sie in DE 101 32 025.6 beschrieben sind.

Weiterhin umfasst die vorliegende Erfindung die Verwendung von nasschemischen Schutzgruppen, z.B. säurelabilen Schutzgruppen wie etwa Tritylschutzgruppen, wobei das zur Abspaltung der Schutzgruppe benötigte Reagenz, z.B. eine Säure wie Trichloressigsäure, in situ durch Belichtung einer Säurevorstufe, z.B. eines Trichloressigsäureesters von substituierten o-Nitrobenzylalkoholen, entsteht. Beispiele für derartige Prozeduren sind von Serafinowski und Garland auf der Konferenz "Chips to Hits 2001 " (IBC's 8th Annual InternationalMicrotechnology Event) vom 28.10. - 01 .1 1.2001 beschrieben worden.

Das erfindungsgemäße Verfahren umfasst die Herstellung eines Trägers mit mehreren, vorzugsweise mit mindestens 50 und besonders bevorzugt mit mindestens 100 verschiedenen Rezeptorbereichen, die mit jeweils unterschiedlichen Analyten in einer einzigen Probe reagieren können. Das erfindungsgemäße Verfahren kann zur Herstellung von Trägern eingesetzt werden, wobei die Rezeptoren in jedem Bereich des Trägers nur eine einzige Sequenz von Bausteinen enthalten. In einer weiteren Ausführungsform kann das erfindungsgemäße Verfahren jedoch auch zur Herstellung von Trägern eingesetzt werden, wobei die Rezeptoren in mindestens einem Bereich des Trägers mehrere verschiedene Sequenzen von Bausteinen enthalten.

Der Start der Synthese kann durch einen nasschemischen oder einen photochemischen Schritt erfolgen.

Im Folgenden sind nun bevorzugte Ausführungsformen des erfindungsgemäßen Verfahren in ausführlicher Form dargestellt:

### Hybridverfahren zur Synthese einer Sequenz pro Stellplatz

Um eine Vielzahl verschiedener Rezeptorsequenzen möglichst effizient auf dem Träger erzeugen zu können, wird anhand eines Computerprogramms die Synthesestrategie (Abfolge von Kondensationsschritten) in Bezug auf einen möglichst hohen Anteil von nasschemischen Synthonbausteinen itteriert. Die Iteration übernimmt ein speziell entwickelter Algorithmus. Als Randbedingung wird der Aspekt, dass alle Sequenzen ortsaufgelöst bereitgestellt werden müssen, berücksichtigt. Hierbei ist es aber nicht notwendig, dass im Falle der Kondensation von nasschemischen Synthonbausteinen, diese sich auf der identischen Syntheseebene befinden müssen. Die Berechnung des kürzesten Syntheseweges erfolgt vorzugsweise nach folgendem Schema:
1. Definition
   Als Syntheseweg wird die Abfolge (mit Wiederholungen) der Nukleotide bezeichnet, die benötigt wird, um alle Rezeptoren des Rezeptorsets vollständig zu erzeugen.
2. Randbedingungen
   Um ein Rezeptorset der Größe s mit maximaler Länge von n Rezeptorbausteinen vollständig erzeugen zu können, sind mindestens n Synthesezyklen notwendig. Dies ist z.B. dann der Fall, wenn alle Rezeptoren die gleiche Sequenz, z.B. Basenabfolge haben. Werden die Rezeptoren schichtweise erzeugt, d.h. müssen alle Rezeptoren die gleiche Länge m erreicht haben, bevor sie auf die Länge m + 1 erweitert werden, werden pro Schicht maximal 4 Zyklen benötigt und somit insgesamt 4ⁿ Zyklen.
   Die Anzahl der Syntheseschritte ist somit immer durch die Länge der Rezeptoren begrenzt und nicht durch die Anzahl der Rezeptoren, die erzeugt werden soll. Es gibt 4" mögliche Reihenfolgen, in denen die Nukleotide gekuppelt werden können, um ein Rezeptorset der Länge n zu erzeugen.
3. Berechnung des kürzesten Wegs
   Um den kürzesten Syntheseweg bestimmen zu können, werden zunächst geeignete Randbedingungen, wie z.B. maximale und minimale Länge des zu berechnenden Wegs bestimmt. Iterativ werden dann alle möglichen Kombinationen durchlaufen und die kürzeste, bzw. bei mehreren kürzesten Wegen eine ausgewählt, die für die Synthese verwendet werden soll. Durch die Wahl von zusätzlichen Randbedingungen kann die Iteration rechtzeitig abgebrochen werden, wenn nicht mehr die Möglichkeit besteht, dass die betrachtete Reihenfolge kürzer wird als die zuvor berechnete.
4. Bestimmung der Entschützungsarten in den einzelnen Syntheseschritten
   Nachdem der kürzeste Syntheseweg bekannt ist, kann nun bestimmt werden, welche Schutzgruppen die einzelnen Bausteine tragen sollen. Jeder Baustein, auf den derjenige Baustein und nur der folgt, der in der Synthesereihenfolge als nächstes gekoppelt werden soll, kann mit einer nasschemischen, z.B. säurelabilen, Schutzgruppe gekoppelt werden. Folgen auf einen gekoppelten Baustein verschiedene Bausteine, so muss er eine photolabile Schutzgruppe tragen, um weiterhin ortsspezifisch synthetisieren zu können.

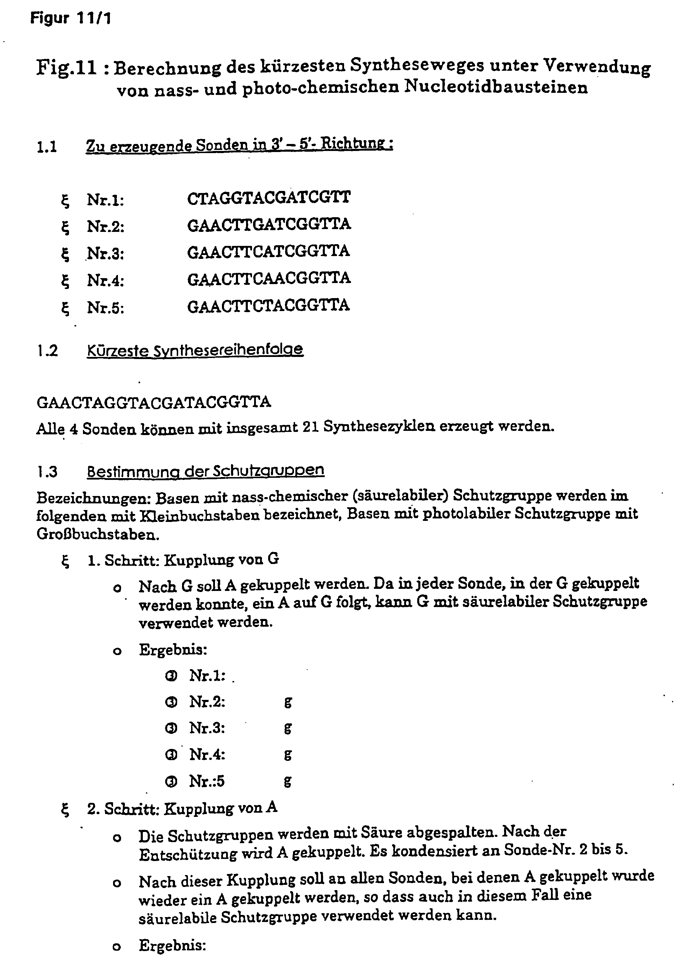

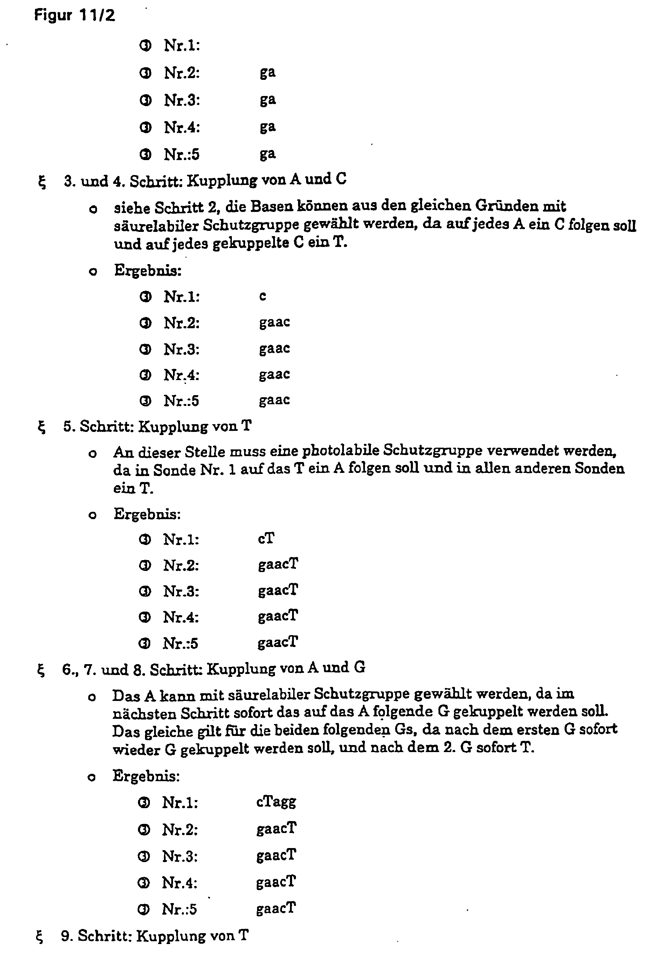

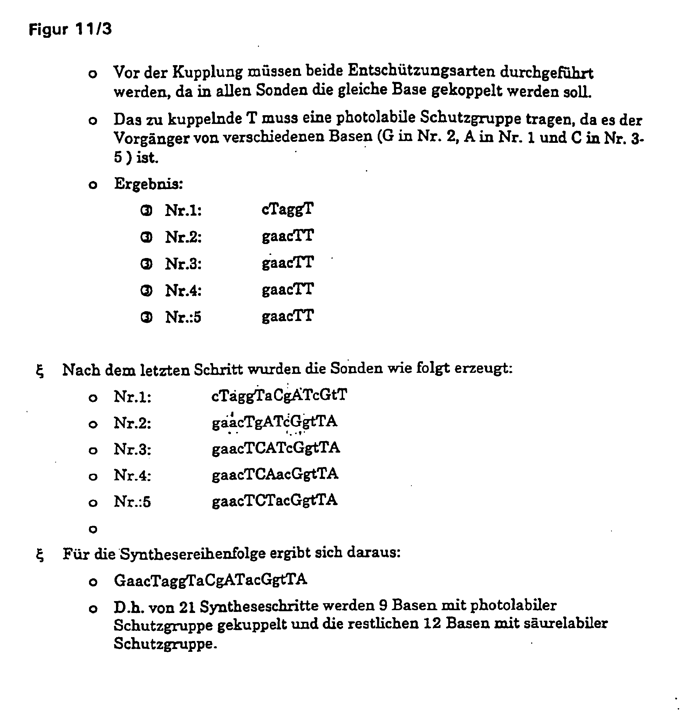

Ein konkretes Ausführungsbeispiel ist in Figur 11 gezeigt.

in einer besonders bevorzugten Ausführungsform wird beim erfindungsgemäßen Hybridverfahren ein mikrofluidischer Reaktionsträger, wie er in der Internationalen Patentanmeldung WO 00/013018 beschrieben ist, verwendet. Hieraus ergibt sich eine zusätzliche räumliche Separation der Syntheseorte, die zu einer Steigerung der Syntheseeffizienz herangezogen werden kann. Hierbei werden bei der coputerunterstützten Optimierung der Synthesestrategie, wobei möglichst viele nasschemische Synthesebausteine verwendet werden sollen, zusätzlich noch die mikrofluidischen Kanäle, in denen Kondensationen gleicher Synthesebausteine zusammengefasst werden, in diese Evaluation mit einbezogen.

Erfindungsgemäß kann nach dem zuvor beschriebenen Hybridverfahren auch die Bereitstellung von mehreren Sequenzen pro Stellplatz erfolgen. Hierbei werden erfindungsgemäß in einer Ausführungsform - zumindest in einem Schritt - nasschemische und photochemische Synthonbausteine gleichzeitig zur Reaktion gebracht.

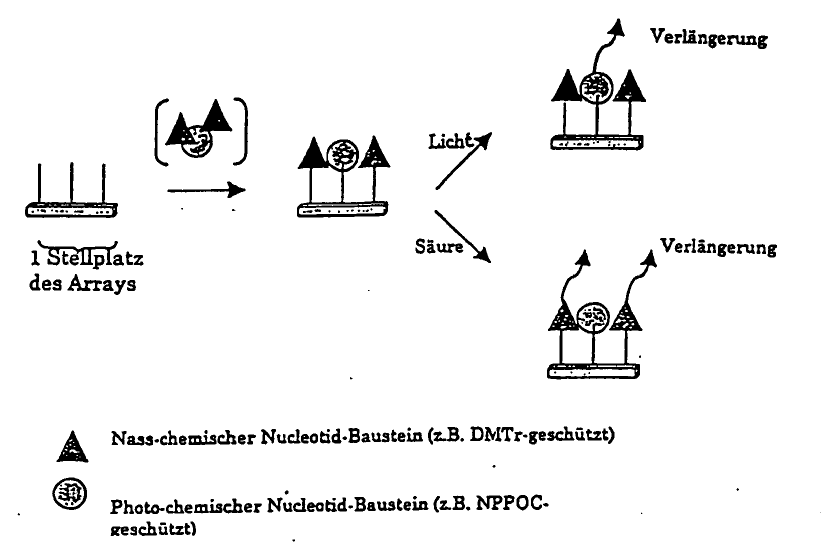

Daraus resultiert, dass ein Anteil der Moleküle pro Stellplatz entweder nach Applikation eines nasschemischen Deprotektionsmediums (z.B. Säure) bzw. ein anderer Anteil nach Applikation eines photochemischen Deprotektionsmediums (Licht) im folgenden synthetisch verlängerbar ist. Beispielhaft seien folgende Synthonbausteine in einer allgemeinen Ausführungsform dargestellt:

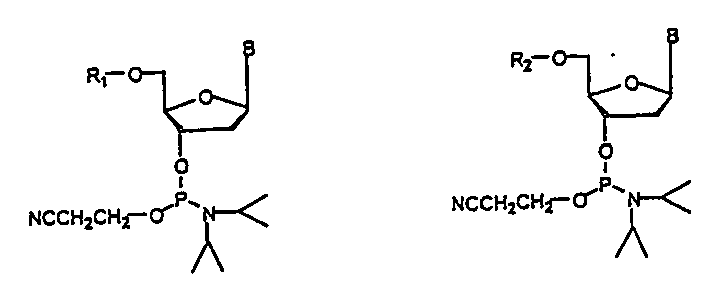

R₁ und R₂ stellen jeweils zueinander orthogonale Schutzgruppen dar, d.h. Schutzgruppen, die unter jeweils verschiedenen Bedingungen abgespalten werden können. Beispiel einer solchen Kombination von R₁ und R₂ sind z.B. DMTr- (säurelabil) und NPPOC-Schutzgruppen (photolabil). Über das Mischungsverhältnis, mit dem die Synthone zur Reaktion gebracht werden, kann - nach nasschemischer bzw. photochemischer Deprotektion - der jeweilige Anteil der im nächsten Schritt verlängerbaren Sonden pro Stellplatz eingestellt werden.

In einer weiteren Ausführungsform wird die Bereitstellung mehrerer Sequenzen pro Stellplatz durch die Verwendung spezieller Hybridbausteine (A), (B) und insbesondere (C), wie sie bereits in den Deutschen Patentanmeldungen DE 100 41 539.3 und DE 100 41 542.3 beschrieben sind, erreicht.

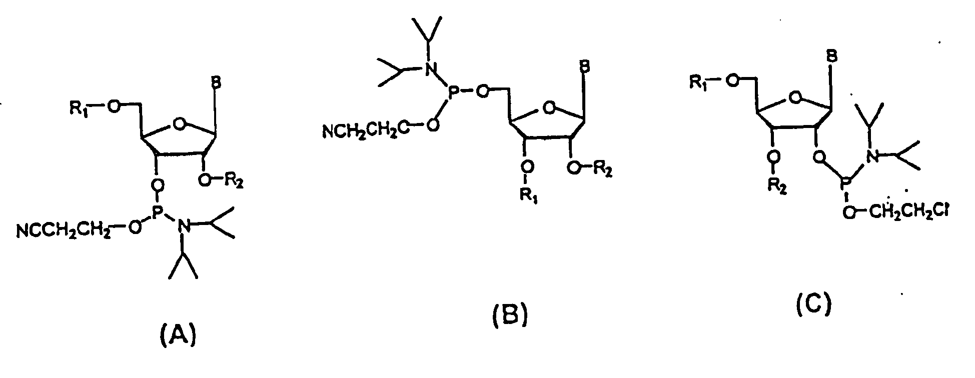

R₁ und R₂ stellen jeweils zueinander orthogonale Schutzgruppen dar. Hierbei erfolgt die Kondensation des hybriden Verzweigungsbausteins mindestens einmal pro Array-Synthese. Erfolgt die Applikation des Verzweigungsbausteins mehrmals während der Array-Synthese, können somit dendrimere Strukturen aufgebaut werden. Im Folgenden ist das Prinzip des hybriden Verzweigungsbausteins dargestellt:

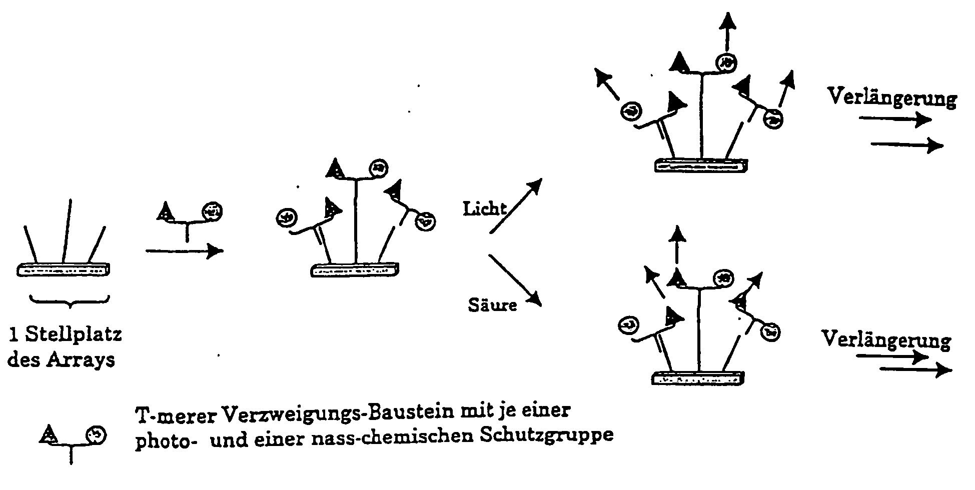

Beim hybriden Verzweigungsbaustein wird das Vorhandensein von nasschemischen und photochemischen Schutzgruppen in einem einzigen Molekül z.B. dazu verwendet, nach nasschemischer Deprotektion an einem Ende des Moleküls die Synthese forzuführen und zu einem späteren Zeitpunkt dann die Fortführung am anderen Ende des Hybridbausteins einzuleiten. Mit diesem Verfahren können pro Stellplatz mehrere Sequenzen aufgebaut werden. Je nach Verwendung von hybriden Verzweigungsbausteinen vom Typ (A), (B) oder (C) erfolgen die Kettenverlängerungen an anderer Stelle:

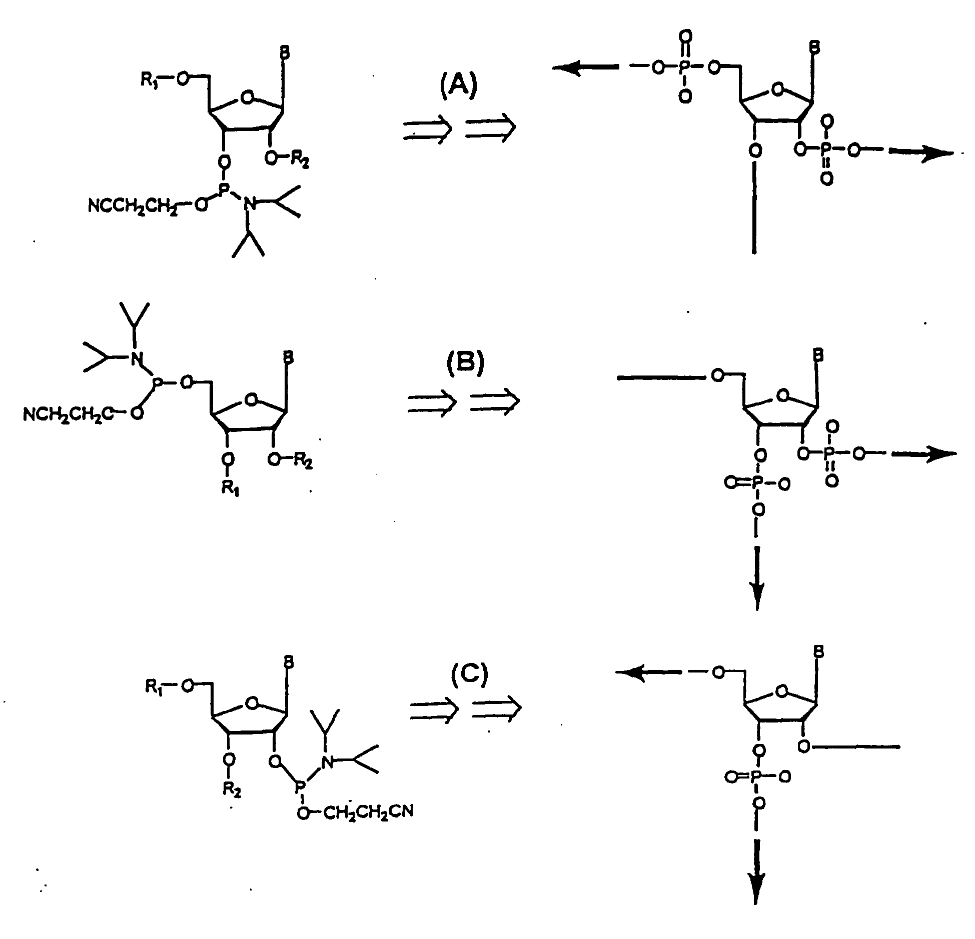

Zur Kettenverlängerung können jegliche Nukleotidbausteine (z.B. 2'-, 3'-, 5'-Phosphitamide) verwendet werden, was zu 3'-5', 5'-3', 5'-5', 3'-3', 2'-2', 5'-2', 3'-2', 2'-3' oder 2'-5' Kettenverlängerungen am Verzweigungsbaustein führen kann.

Eine konkrete Vorgehensweise ist im Folgenden am Beispiel eines hybriden Verzweigungsbausteins vom Typ (A) dargestellt. Nach Abspaltung von Schutzgruppe R₁ (z.B. photolabil) erfolgt die Kettenverlängerung am 5'-Ende des Verzweigungsbausteins und die Sequenz 1 wird erzeugt. Nach Vervollständigung der Sequenz 1 wird durch einen Capping-Schritt ein Weiterwachsen verhindert. Wird dann die R₂-Schutzgruppe (z.B. säurelabil) entfernt, kann am 3'-Ende die Kettenverlängerung erfolgen, bis dann Sequenz 2 aufgebaut ist. Sequenz 1 und 2 können, aber müssen nicht komplementär zu der gleichen in der zu untersuchenden Probe befindlichen Target-Sequenz sein.

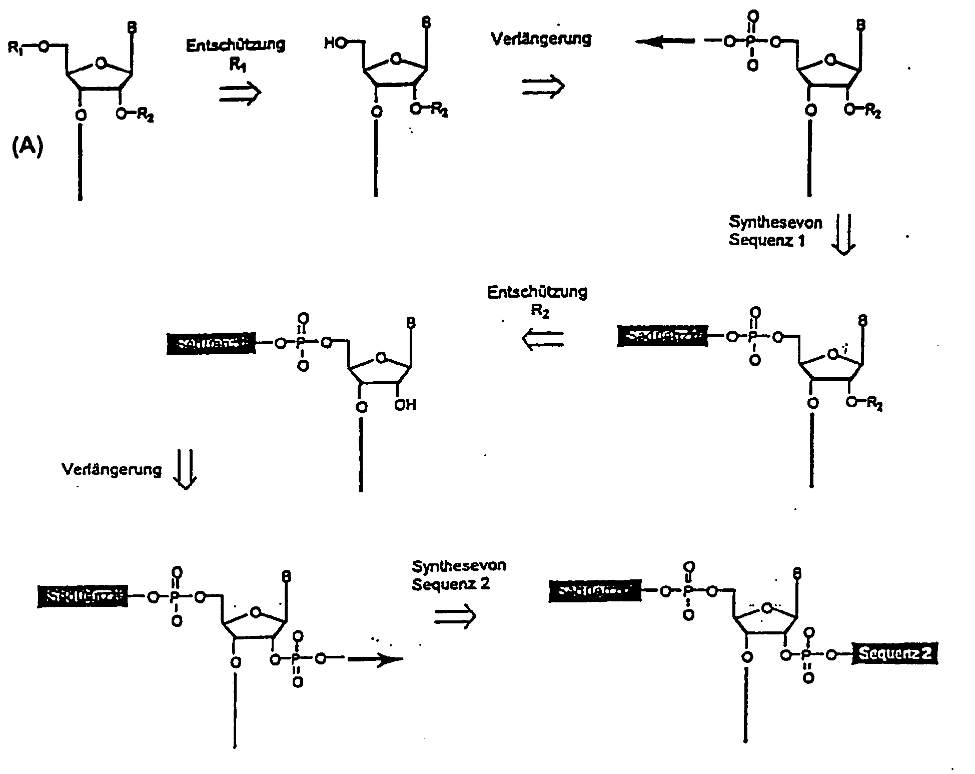

Wird z.B als zweite Sequenz bei allen Stellplätzen die gleiche Sequenz neben den für das eigentliche Hybridisierungsexperiment notwendigen Sonden aufgebaut, kann diese Sequenz als Referenzsequenz dienen.

Dadurch lässt sich der Micro-Array sehr genau normieren, da für jeden Stellplatz eine Kontrollsequenz vorhanden ist.

Um das Entstehen von erhöhten Hintergrundsignalen durch die Referenzsonden zu vermeiden, muss sichergestellt werden, dass diese Sequenzen nicht in der zu untersuchenden Probe enthalten sind. Dies wird erfindungsgemäß dadurch erreicht, dass dies im Vorfeld der Array-Herstellung erfolgt oder/und dass zur Erzeugung der Referenzsonden besondere Bausteine von Nukleinsäureanaloga verwendet werden, die nicht mit DNA-Molekülen paaren (Beier et al., Science 1999, Vol. 283, 69-703).

Zur Analyse werden dann entweder simultan (z.B. mit 2 Farben-Detektion) oder getrennt die zu untersuchenden DNA-Targets und die entsprechenden komplementären Referenzsequenzen - die nur mit ihresgleichen und nicht mit der zu untersuchenden DNA paaren - auf dem Micro-Array hybridisiert. So kann für jeden Stellplatz des Arrays ein individueller Abgleich des Hybridisierungssignals der zu untersuchenden Probesequenz mit dem Signal, der sich auf dem gleichen Stellplatz befindlichen Referenzsequenz, erfolgen. Hierdurch können herstellungsbedingte (ungleichmäßige Belichtung, ungleichmäßige Derivatisierung) bzw. hybridisierungsbedingte (Fussel, Reflexionen) Unregelmäßigkeiten des Arrays herausgemittelt werden.

In einer weiteren Ausführungsform können hybride Verzweigungsbausteine in Enzym-Reaktionen eingesetzt werden. So kann z.B. ein hybrider Verzweigungsbaustein als Primer für eine Polymerase-Reaktion oder auch für eine Ligase-Reaktion verwendet werden.

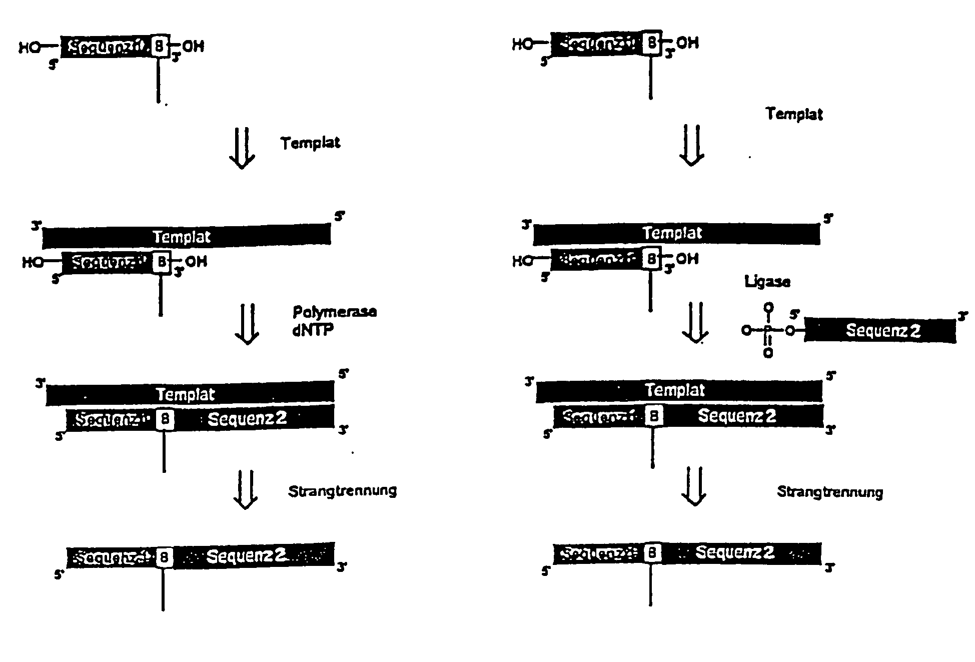

Hierbei muss die für das Enzym notwendige Anknüpfungsstelle nicht notwendigerweise (wie oben abgebildet) direkt am hybriden Verknüpfungsbaustein liegen. Es können durchaus mehrere Nukleotidbausteine zwischen Verknüpfungsbausteinen und Anknüpfungsstelle des Enzyms liegen.

Die vorliegende Erfindung ermöglicht erhebliche Verbesserungen gegenüber den bekannten Verfahren zur Synthese von Rezeptorarrays, da durch das Hybridverfahren eine computeroptimierte Synthesestrategie für die ortsaufgelöste in situ Synthese eingesetzt werden kann. Dabei werden möglichst viele nasschemische Synthonbausteine verwendet, um eine höhere Qualität der Syntheseprodukte zu erreichen. Desweiteren erlaubt die Verwendung möglichst vieler nasschemischer Bausteine auch eine Steigerung der Synthesegeschwindigkeit, ohne dadurch in der Flexibilität der ortsaufgelösten Synthese eingeschränkt zu werden. Die Optimierung des Synthesewegs kann durch einen spezifischen Algorithmus, wie zuvor angegeben, berechnet werden.

Durch Kombination nasschemischer und photochemischer Bausteine kann bei der in situ Synthese von Rezeptorarrays hochparallel eine hohe Anzahl verschiedener Rezeptoren erzeugt werden. Dies kann noch weiter gesteigert werden, wenn ein mikrofluidischer Reaktionsträger, z.B. mit einer Vielzahl paralleler Kanäle verwendet wird.

Weiterhin kann durch parallele Kondensation von nasschemischen und photochemischen Rezeptorbausteinen gezielt der Aufbau von mehreren Rezeptorsequenzen pro Stellplatz bzw. Bereich erfolgen. Dies kann auch durch Verwendung von Hybridbausteinen, die sowohl eine nasschemische als auch eine photochemische Schutzgruppe tragen, erfolgen. So kann z.B. eine Sequenz pro Stellplatz als Referenz (Qualitätskontrolle) dienen, während eine andere für das eigentliche Experiment zur Verfügung steht.

Die genannten Verbesserungen können durch ein reines nass- bzw. photochemisches Verfahren zur Herstellung des Trägers allein nicht erreicht werden.

Weiterhin soll die vorliegende Erfindung durch die folgenden Abbildungen erläutert werden.

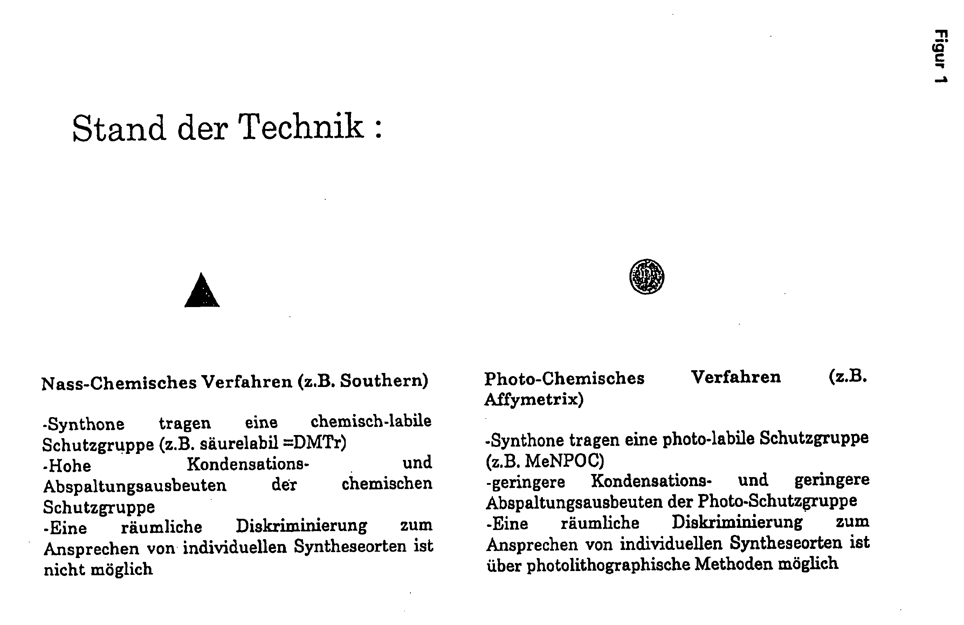

- **Figur 1**: zeigt eine Gegenüberstellung von nasschemischen bzw. photochemischen Verfahren zur Erzeugung von DNA-Arrays mittels in situ Synthese (Stand der Technik)

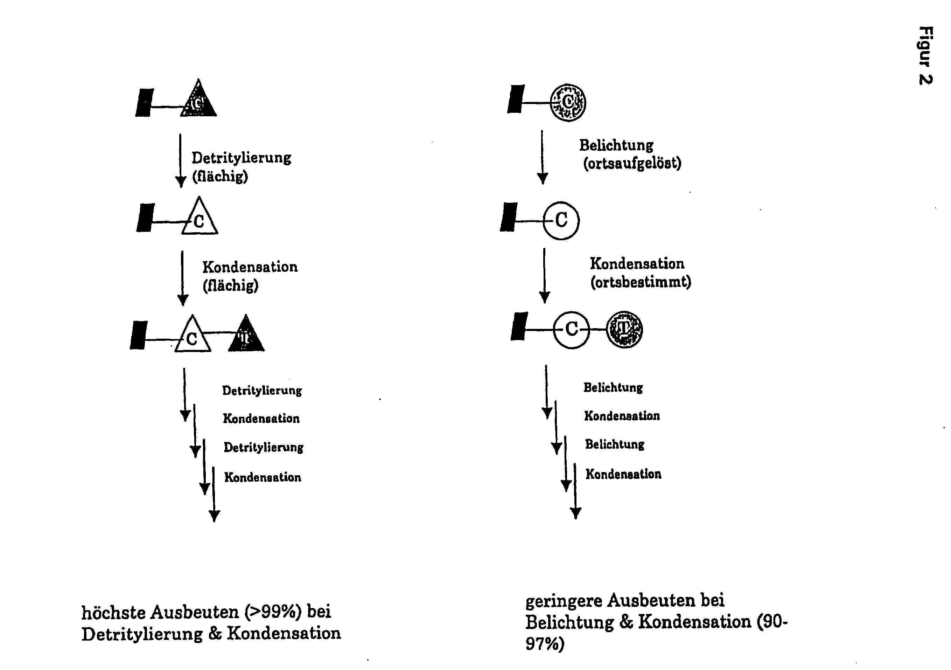

- **Figur 2**: zeigt eine Gegenüberstellung der Reaktionsabläufe bei nasschemischen und photochemischen Verfahren (Stand der Technik)

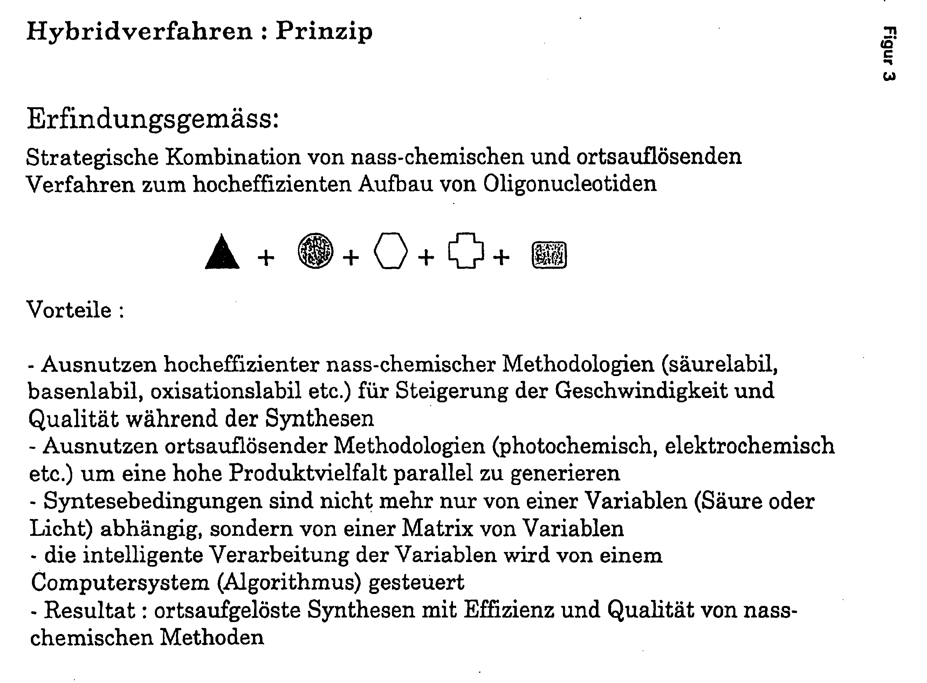

- **Figur 3**: zeigt das Prinzip des Hybridverfahrens unter Synthese einer Sequenz pro Stellplatz

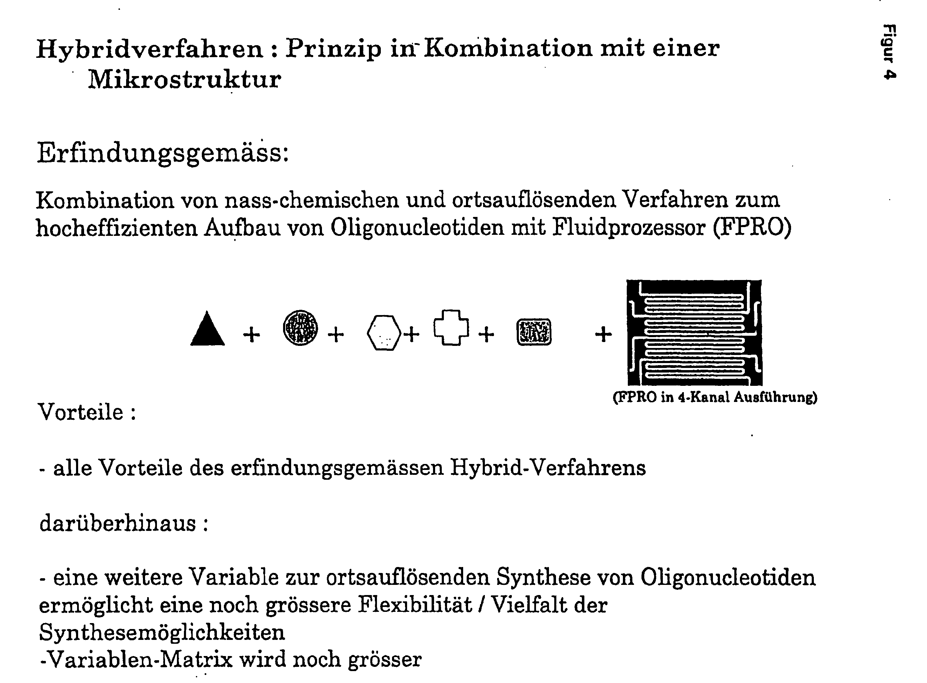

- **Figur 4**: zeigt das Prinzip des Hybridverfahrens unter Synthese einer Sequenz pro Stellplatz unter Verwendung eines mikrofluidischen Reaktionsträgers

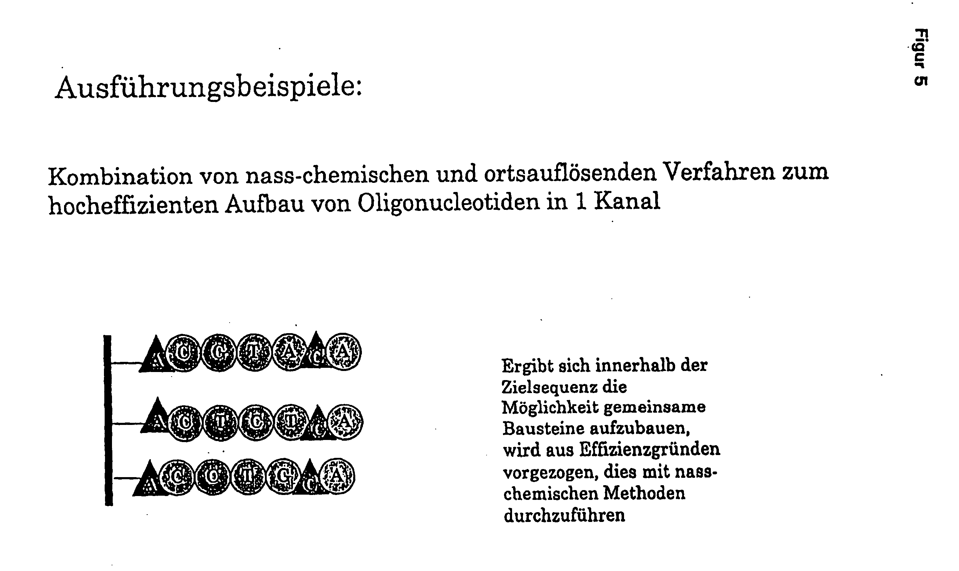

- **Figur 5**: zeigt eine Ausführungsform der erfindungsgemäßen Kombination von nasschemischen und photochemischen Verfahren; die gemeinsam kondensierten nasschemischen Bausteine befinden sich auf einer Syntheseebene

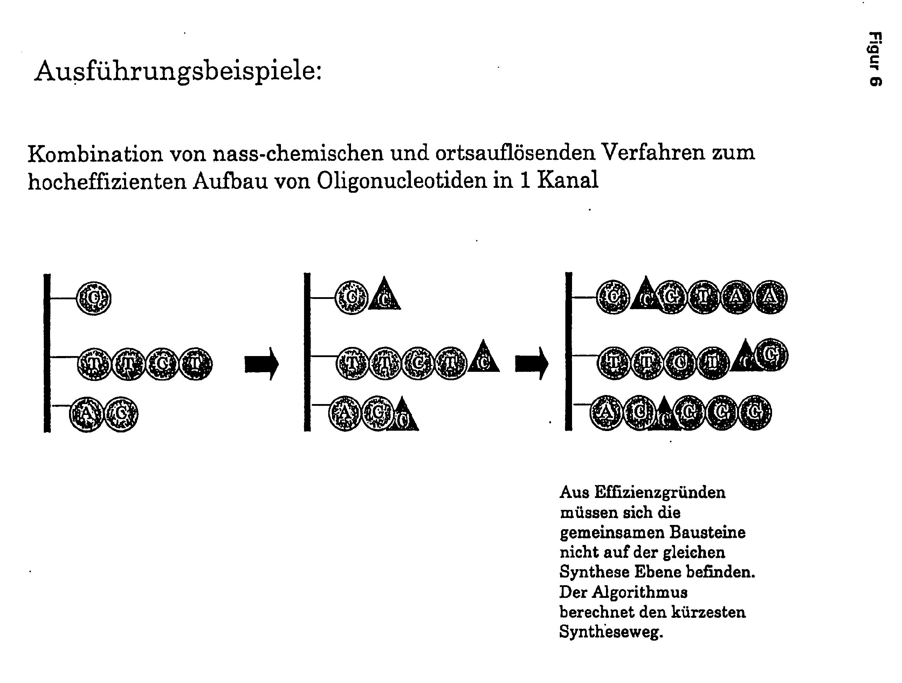

- **Figur 6**: zeigt eine weitere Ausführungsform der erfindungsgemäßen Kombination von nasschemischen und photochemischen Verfahren; die gemeinsam kondensierten nasschemischen Bausteine befinden sich nicht auf einer Syntheseebene

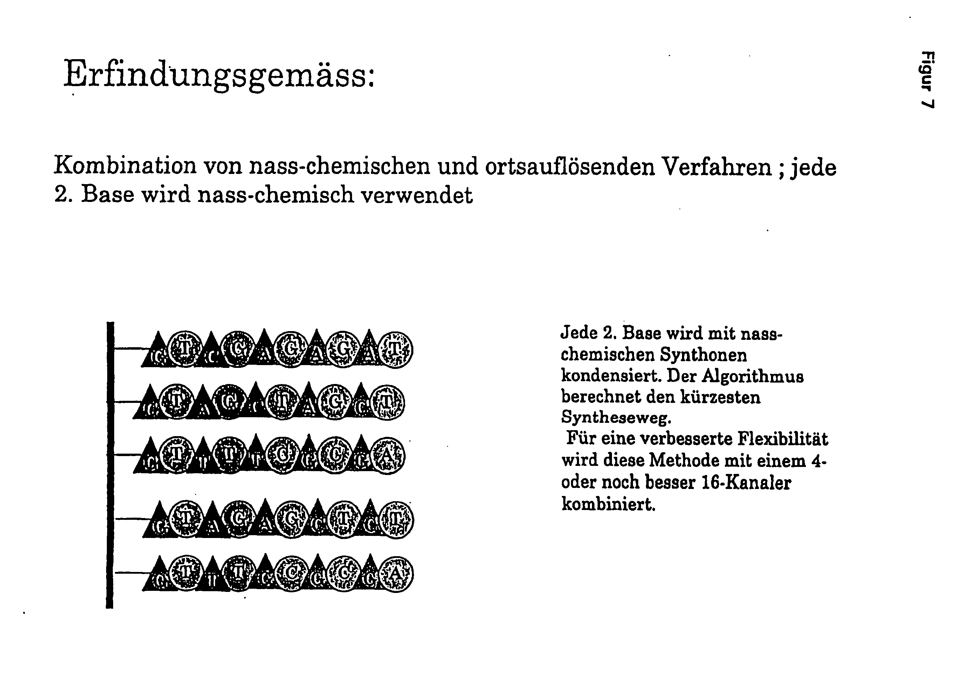

- **Figur 7**: zeigt eine weitere Ausführungsform der erfindungsgemäßen Kombination von nasschemischen und photochemischen Verfahren; jede 2. Base wird nasschemisch kondensiert

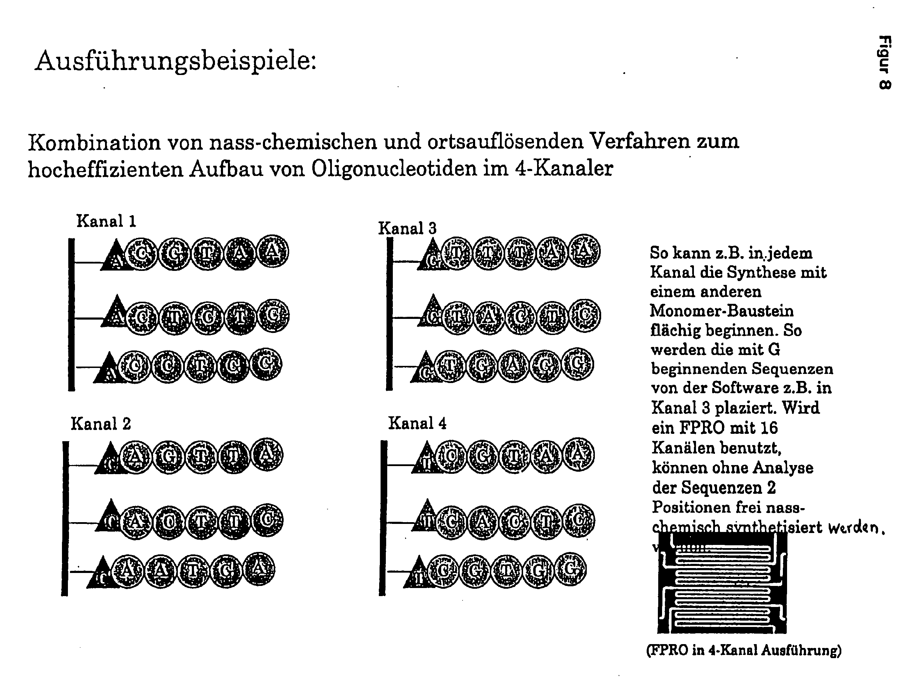

- **Figur 8**: zeigt eine weitere Ausführungsform der erfindungsgemäßen Kombination von nasschemischen und photochemischen Verfahren in Kombination mit einem mikrofluidischen Reaktionsträger mit 4 Kanälen

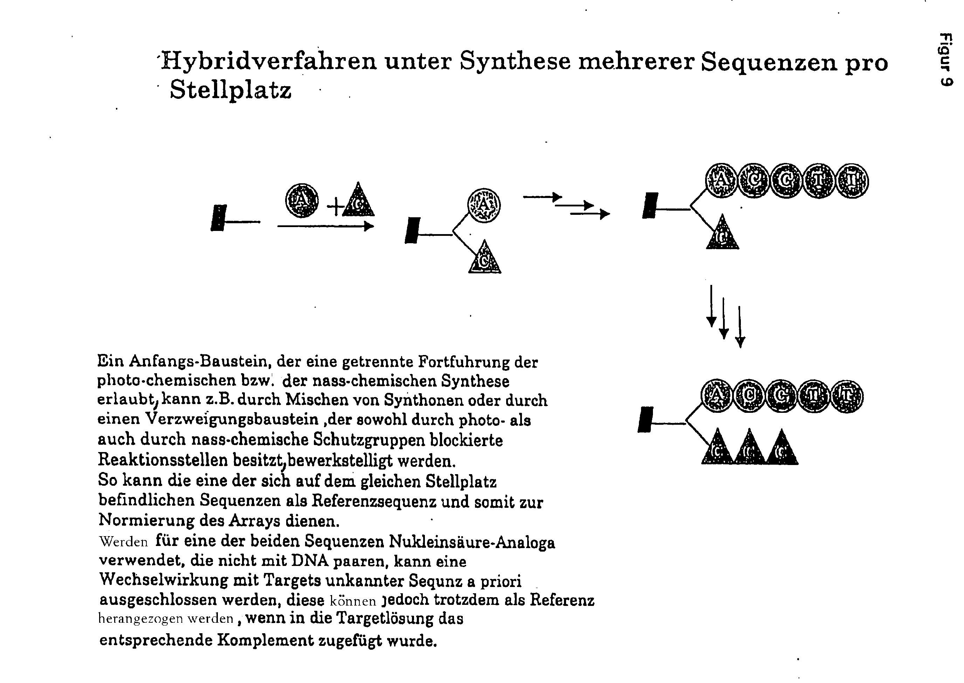

- **Figur 9**: zeigt das Prinzip des Hybridverfahrens unter Synthese mehrerer Sequenzen pro Stellplatz mit Hilfe eines Gemisches von Nukleotidbausteinen

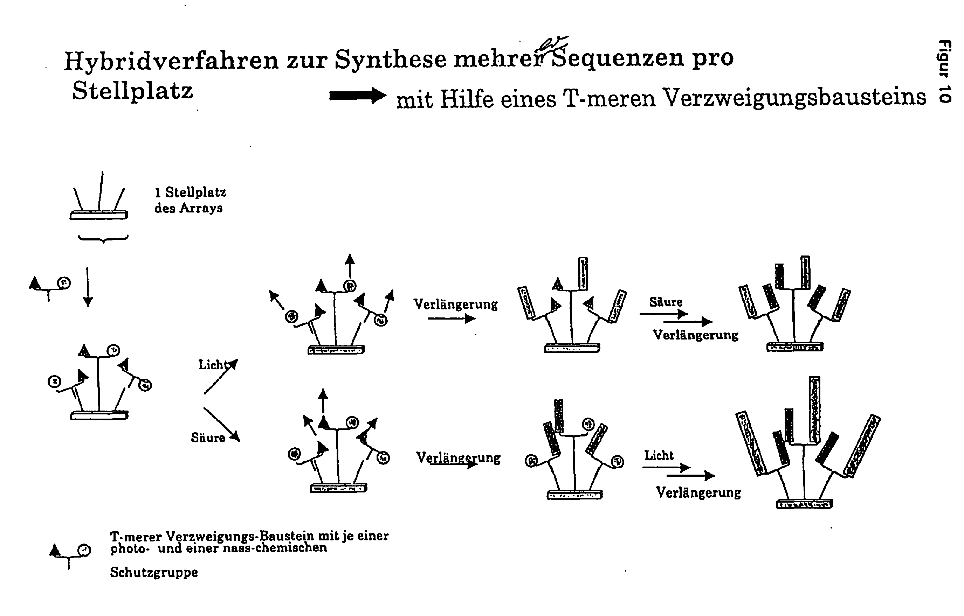

- **Figur 10**: zeigt das Prinzip des Hybridverfahrens unter Synthese mehrerer Sequenzen pro Stellplatz mit Hilfe eines T-meren Verzweigungsbausteins
- **Figur 11**: zeigt die Berechnung des kürzesten Syntheseweges unter Verwendung von nass- und photochemischen Nukleotidbausteinen

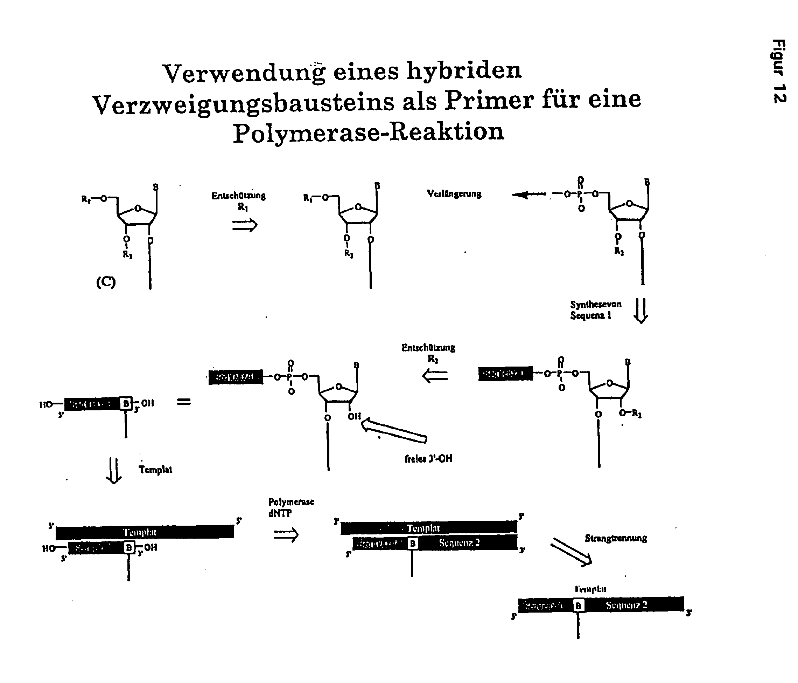

- **Figur 12**: zeigt die Verwendung eines hybriden Verzweigungsbaustein als Primer für eine Polymerasereaktion

## Patentansprüche

1. Verfahren zur Herstellung eines Trägers für die Bestimmung von Analyten, umfassend die Schritte:
**(a) Bereitstellen eines Trägers,**
(b) Leiten von Flüssigkeit mit Bausteinen für die Synthese polymerer Rezeptoren über den Träger,
(c) orts- oder/und zeitspezifisches Immobilisieren der Rezeptorbausteine an jeweils vorbestimmten Bereichen auf dem Träger und
(d) Wiederholen der Schritte (b) und (c) bis die gewünschten Rezeptoren an den jeweils vorbestimmten Bereichen synthetisiert worden sind,
**dadurch gekennzeichnet,**
**dass** die Synthese der Rezeptoren eine Kombination von nasschemischen und photochemischen Syntheseschritten umfasst und dass man einen Träger mit mehreren verschiedenen Rezeptorbereichen herstellt.

2. Verfahren nach Anspruch 1,
**dadurch gekennzeichnet,**
**dass** man einen mikrofluidischen Träger mit Kanälen, vorzugsweise mit geschlossenen Kanälen verwendet, in denen vorbestimmte Bereiche mit immobilisierten Rezeptoren entstehen.

3. Verfahren nach Anspruch 1 oder 2,
**dadurch gekennzeichnet,**
**dass** die Rezeptoren ausgewählt werden aus Biopolymeren wie etwa Nukleinsäuren, Nukleinsäureanaloga, Proteinen, Peptiden und Kohlenhydraten.

4. Verfahren nach einem der Ansprüche 1 bis 3,
**dadurch gekennzeichnet,**
**dass** die Rezeptoren aus Nukleinsäuren und Nukleinsäureanaloga ausgewählt werden.

5. Verfahren nach Anspruch 4,
**dadurch gekennzeichnet,**
**dass** die Synthese der Rezeptoren die Verwendung einer Kombination von nasschemischen Phosphoramidit-Bausteinen und von photochemischen Phosphoramidit-Bausteinen umfasst.

6. Verfahren nach einem der Ansprüche 1 bis 5,
**dadurch gekennzeichnet,**
**dass** man einen Träger mit mindestens 50, vorzugsweise mit mindestens 100 verschiedenen Rezeptorbereichen herstellt.

7. Verfahren nach einem der Ansprüche 1 bis 6,
**dadurch gekennzeichnet,**
**dass** die Rezeptoren in einem Bereich des Trägers eine einzige Sequenz von Bausteinen enthalten.

8. Verfahren nach einem der Ansprüche 1 bis 6,
**dadurch gekennzeichnet,**
**dass** die Rezeptoren in einem Bereich des Trägers mehrere verschiedene Sequenzen von Bausteinen enthalten.

9. Verfahren nach Anspruch 8,
**dadurch gekennzeichnet,**
**dass** die Rezeptoren in einem Bereich des Trägers aus Referenzsequenzen und Analytbestimmungssequenzen bestehen.

10. Verfahren nach einem der Ansprüche 1 bis 9,
**dadurch gekennzeichnet,**
**dass** Synthesebausteine verwendet werden, die eine nasschemische und eine photochemische Schutzgruppe tragen.

11. Verfahren nach einem der Ansprüche 1 bis 10,
**dadurch gekennzeichnet,**
**dass** man den Träger in situ für einen Analytbestimmungsprozess verwendet.

## Revendications

1. Procédé de préparation d'un support pour la détermination d'analyte, comprenant les étapes de :
(a) disposition d'un support,
(b) passage d'un liquide avec les éléments pour la synthèse des récepteurs polymères sur le support,
(c) immobilisation spécifique de site et/ou de moment des éléments de récepteur sur chaque zone prédéterminée sur le support, et
(d) répétition des étapes (b) et (c) jusqu'à ce que les récepteurs souhaités ont été synthétisés en chaque zone prédéterminée,
**caractérisé en ce que** la synthèse des récepteurs comprend une combinaison d'étapes de synthèse de chimie humide et de photochimie et **en ce que** l'on prépare un support avec plusieurs zones différentes de récepteur.

2. Procédé selon la revendication 1, **caractérisé en ce que** l'on utilise un support microfluide avec des canaux, de préférence avec canaux fermés, dans lesquels des zones prédéterminées avec des récepteurs immobilisés se forment.

3. Procédé selon la revendication 1 ou 2, **caractérisé en ce que** les récepteurs sont choisis parmi des biopolymères, comme des acides nucléiques, des analogues d'acide nucléique, des protéines, des peptides et des hydrates de carbone.

4. Procédé selon l'une des revendications 1 à 3, **caractérisé en ce que** les récepteurs sont choisis parmi des acides nucléiques et des analogues d'acide nucléique.

5. Procédé selon la revendication 4, **caractérisé en ce que** la synthèse des récepteurs comprend l'utilisation d'une combinaison d'éléments phosphoramidite de chimie humide et d'éléments phosphoramidite de photochimie.

6. Procédé selon l'une des revendications 1 à 5, **caractérisé en ce que** l'on prépare un support avec au moins 50, de préférence au moins 100 zones différentes de récepteur.

7. Procédé selon l'une des revendications 1 à 6, **caractérisé en ce que** les récepteurs dans une zone du support, contiennent une séquence unique des éléments.

8. Procédé selon l'une des revendications 1 à 6, **caractérisé en ce que** les récepteurs dans une zone du support, contiennent plusieurs séquences différentes des éléments.

9. Procédé selon la revendication 8, **caractérisé en ce que** les récepteurs dans une zone du support consistent en des séquences de référence et des séquences de détermination d'analyte.

10. Procédé selon l'une des revendications 1 à 9, **caractérisé en ce que** l'on utilise des éléments de synthèse, qui portent un groupe protecteur de chimie humide et un groupe protecteur de photochimie.

11. Procédé selon l'une des revendications 1 à 10, **caractérisé en ce que** l'on utilise le support *in situ,* pour un processus de détermination d'analyte.

## Claims

1. A method for producing a carrier for the determination of analytes, comprising the steps:
(a) providing a carrier,
(b) conducting a liquid containing building blocks for the synthesis of polymeric receptors over the carrier,
(c) immobilizing in a location- or/and time-specific manner the receptor building blocks in in each case predetermined regions on said carrier and
(d) repeating steps (b) and (c), until the desired receptors have been synthesized in the in each case predetermined regions,
**characterized in that**
synthesis of said receptors comprises a combination of wet-chemical and photochemical synthesis steps and that a carrier with a plurality of different receptor regions is produced.

2. The method as claimed in claim 1,
**characterized in that**
a microfluidic carrier with channels, preferably with closed channels, in which predetermined regions with immobilized receptors are generated, is used.

3. The method as claimed in claim 1 or 2,
**characterized in that**
the receptors are selected from biopolymers such as, for example, nucleic acids, nucleic acid analogs, proteins, peptides and carbohydrates.

4. The method as claimed in any of claims 1 to 3,
**characterized in that**
the receptors are selected from nucleic acids and nucleic acid analogs.

5. The method as claimed in claim 4,
**characterized in that**
synthesis of the receptors comprises using a combination of wet-chemical phosphoramidite building blocks and of photochemical phosphoramidite building blocks.

6. The method as claimed in any of claims 1 to 5,
**characterized in that**
a carrier with at least 50, and preferably with at least 100, different receptor regions is produced.

7. The method as claimed in any of claims 1 to 6,
**characterized in that**
the receptors contain in one region of the carrier a single sequence of building blocks.

8. The method as claimed in any of claims 1 to 6,
**characterized in that**
the receptors in one region of the carrier contain a plurality of different sequences of building blocks.

9. The method as claimed in claim 8,
**characterized in that**
the receptors in one region of the carrier comprise reference sequences and analyte determination sequences.

10. The method as claimed in any of claims 1 to 9,
**characterized in that**
synthetic building blocks are used which carry a wet-chemical and a photochemical protective group.

11. The method as claimed in any of claims 1 to 10,
**characterized in that**
the carrier is used in situ for an analyte determination process.
